(19) Europäisches Patentamt
European Patent Office
Office européen des brevets

(11) Veröffentlichungsnummer **0 003 286**
**B1**

(12) # EUROPÄISCHE PATENTSCHRIFT

(45) Veröffentlichungstag der Patentschrift: **24.03.82**

(21) Anmeldenummer: **79100060.7**

(22) Anmeldetag: **10.01.79**

(51) Int. Cl.³: **C 07 D 519/02,**
C 07 D 498/14,
A 61 K 31/48 //C07D487/04,
(C07D498/14, 263/00,
241/00, 209/00)

(54) **Ergopeptidalkaloid-Derivate, Verfahren zu ihrer Herstellung und diese enthaltende pharmazeutische Zusammensetzungen.**

(30) Priorität: **20.01.78 CH 622/78**

(43) Veröffentlichungstag der Anmeldung:
**08.08.79 Patentblatt 79/16**

(45) Bekanntmachung des Hinweises auf die Patenterteilung:
**24.03.82 Patentblatt 82/12**

(84) Benannte Vertragsstaaten:
**BE CH DE FR GB LU NL SE**

(56) Entgegenhaltungen:
**DE - A - 1 620 386**
**DE - A - 2 431 963**
**DE - A - 2 700 234**

(73) Patentinhaber: **SANDOZ AG**
**Lichtstrasse 35**
**CH-4002 Basel (CH)**

(72) Erfinder: **Stadler, Paul, Dr.**
**Jakobsweg 7**
**CH-4105 Biel-Benken (CH)**
Erfinder: **Troxler, Franz, Dr.**
**Drosselstrasse 39**
**CH-4103 Bottmingen (CH)**

**EP 0 003 286 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Ergopeptidalkaloid-Derivate, Verfahren zu ihrer Herstellung und diese enthaltende pharmazeutische Zusammensetzungen

Die vorliegende Erfindung betrifft Ergopeptidalkaloid-Derivate, Verfahren zu ihrer Herstellung und diese enthaltenden pharmazeutischen Zusammensetzungen.

Die Erfindung betrifft eine Verbindungen der Formel I,

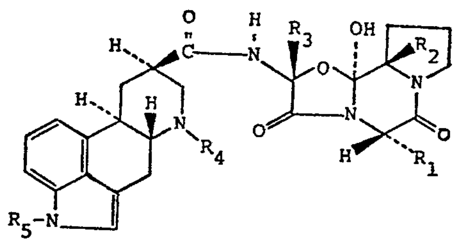

I

worin $R_1$ gegebenenfalls durch Hydroxy, Chlor, Fluor, Carbamoyl, Amino, Alkanoylamino mit 2 bis 5 Kohlenstoffatomen, Alkyl, Alkylamino, Alkylcarbamoyl oder Alkoxy mit 1 bis 4 Kohlenstoffatomen, Dialkylcarbamoyl oder Dialkylamino, wobei jede Alkylgruppe der beiden letzten Radikale 1 bis 4 Kohlenstoffatome enthält, substituiertes Benzyl, $R_2$, $R_4$ und $R_5$ Alkyl mit 1 bis 4 Kohlenstoffatomen und $R_3$ verzweigtes Alkyl mit 3 bis 4 Kohlenstoffatomen bedeuten.

Alkanoyl hat üblicherweise 2 Kohlenstoffatome. Jede andere Gruppe mit 1 bis 4 Kohlenstoffatomen hat üblicherweise 1 Kohlenstoffatom. $R_3$ steht beispielsweise für Isopropyl. $R_1$ steht üblicherweise für Benzyl, das monosubstituiert sein kann und ist vorzugsweise Benzyl.

Die DE—A 2 431 963 und 2 700 234 betreffen Herstellungsverfahren von Verbindungen aus der Klasse der Ergotpeptidalkaloide. In der DE—A 1 620 386 werden gewisse Ergopeptidalkaloid-Derivate beschrieben, die anstelle der verzweigten Alkylgruppe $R_3$ eine Methylgruppe aufweisen. Laut dieser Anmeldung zeichnen sich diese 2'-Methylderivate durch eine adrenolytische und Blutdruckbeeinflussende Wirksung aus. Bestimmte Untergruppen dieser Verbindungsklasse weisen überdies eine Antiserotonin- bzw. uterotonische Wirkung auf.

Die vorliegende Erfindung umfasst auch Verfahren zur Herstellung einer Verbindung der Formel I, indem man ein Säureadditionssalz einer Verbindung der Formel II,

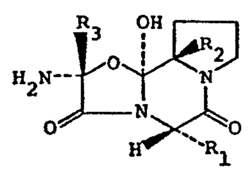

II

worin R1, $R_2$ und $R_3$ obige Bedeutung haben, mit einem reaktiven Säurederivat einer Verbindung der Formel III,

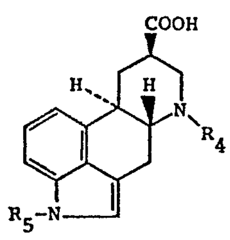

III

worin R4 und $R_5$ obige Bedeutung haben, kondensiert.

Das Verfahren kann analog zu bekannten Methoden für die Kondensation zur Herstellung von analogen cyclischen Ergotpeptidalkaloiden erfolgen.

Das reaktive Säurederivat einer Verbindung der Formel III kann beispielsweise in situ gebildet werden. Vorzugsweise verwendet man das Additionsprodukt einer Verbindung der Formel III mit Dimethylformamid oder Dimethylacetamid und Oxalylchlorid. Vorzugsweise wird die Reaktion in Gegenwart von Triäthylamin oder Pyridin durchegeführt. Geeignete Lösungsmittel sind z.B. Chloroform, Methylenchlorid, Dimethylformamid oder Acetonitril.

Die Umsetzung kann bei Temperaturen zwischen —30° bis +20°C durchgeführt werden.

Die Verbindungen der Formel II sind neu und können hergestellt werden, indem man ein Säureadditionssalz einer Verbindung der Formel II durch Abspaltung der Schutzgruppe aus einer Verbindung der Formel IV,

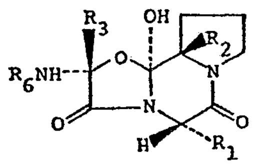

IV

worin $R_1$, $R_2$ und $R_3$ obige Bedeutung haben und $R_6$ eine Schutzgruppe bedeutet, in Gegenwart einer Säure gewinnt. Das Verfahren kann analog zu bekannten Methoden für die Abspaltung von Schutzgruppen aus analogen basenempfindlichen Peptiden, beispielsweise mittels Hydrogenlolyse. Vorzugsweise ist die Schutzgruppe die Benzyloxycarbonylgruppe. Zweckmässig verwendet man als Säure eine Mineralsäure wie HCl.

Die Verbindung der Formel VI, worin $R_6$=Benzyloxycarbonyl bedeutet, kann hergestellt werden, indem man aus einer Verbindung der Formel V,

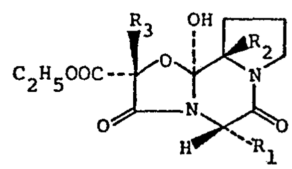

V

worin $R_1$, $R_2$ und $R_3$ obige Bedeutung haben, in an sich bekannter Weise zuerst die entsprechende Säure bildet, hierauf über das Säurechlorid und über das Säureazid in die entsprechende Benzyloxycarbonylamino-Verbindung überführt.

Eine Verbindung der Formel V kann hergestellt werden durch Reaktion einer Verbindung der Formel VI

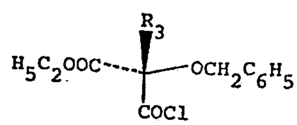

VI

mit einer Verbindung der Formel VII

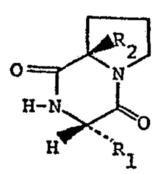

VII

und anschliessender Hydrierung des entstehenden Produktes der Formel VIII.

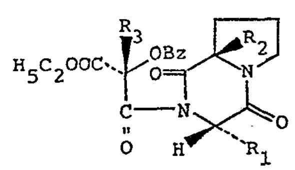

VIII

Insoweit die Herstellung der Ausgangsverbindungen nicht besonders beschrieben wurde, sind diese bekannt oder können nach an sich bekannten Methoden, z.B. für die Herstellung von Peptiden, hergestellt werden.

Aus den freien Basen der Verbindungen der Formel I lassen sich in bekannter Weise Säureadditionssalze herstellen und umgekehrt. Geeignete Salze schliessen das Hydrochlorid und das Methansulfonat ein.

Die Verbindungen der Formel I, d.h. die Verbindungen der Formel I in freier Form oder in Form ihrer physiologisch verträglichen Salze, zeichnen sich durch interessante pharmakodynamische Eigenschaften aus. Sie können als Heilmittel verwendet werden.

Sie bewirken eine Stimulation der Prolaktinsekretion. Die Prolaktin-sekretionsfördernde Wirkung konnte an der männlichen Ratte in Dosen ab 0,1 bis 10 mg/kg s.c. mittels RIA-Methodik durch Analyse

der Serum-Prolaktinkonzentration nachgewiesen werden. Auch bei der weiblichen adulten Ratte konnte Induktion von Pseudogravidität als Indikator einer vermehrten Prolactinsekretion in Dosen von 0,25 bis 5,0 mg/kg s.c. nachgewiesen werden. Die Verbindungen der Formel I können daher bei der Fertilitätshemmung Verwendung finden.

Bei der weiblichen juvenilen Ratte (ca. 40g) konnte eine Induktion der Pubertät in Dosen von 2 × 3 mg/Tier oral pro Tag nach 10-tägiger Behandlung nachgewiesen werden. Die Verbindungen der Formel I können daher bei der Behandlung der Pubertas tarda Verwendung finden.

Bei weiblichen adulten Ratten konnte eine mammotrope Wirkung bei mit Oestrogen vorbehandelten Tieren in Dosen von 2 × 3 mg/kg s.c. pro Tag nach 5 Tagen nachgewiesen werden. Die Verbindungen der Formel I können daher bei mangelhafter Milchbildung Verwendung finden.

Für obige Anwendung liegt die Tagedosis bei etwa 5 bis 100 mg. Diese Dosis kann gegebenenfalls auch in kleineren Dosen 2 bis 4 mit täglich oder in Retardform verabreicht werden. Eine Einheitsdosis, beispielsweise eine zur oralen Verabreichung geeignete Tablette, kann zwischen 1 und 50 mg des Wirkstoffes zusammmen mit geeigneten pharmazeutisch indifferenten Hilfsstoffen enthalten.

Die Verbindungen der Formel I in freier Form oder in Form ihrer physiologisch verträglichen Salze können allein oder in geeigneter Dosierungsform verabreicht werden. Die Arzneiformen, z.B. eine Tablette, können analog zu bekannten Methoden hergestellt werden.

Gegenstand der Erfindung sind daher auch Heilmittel, die die erfindungsgemässen Verbindungen enthalten, sowie die Herstellung dieser Heilmittel auf an sich bekannte Weise. Für Ihre Herstellung können die in der Pharmazie gebräuchlichen Hilfs- und Trägerstoffe verwendet werden.

In einer Verbindungsgruppe steht $R_1$ für Benzyl oder durch Hydroxy, Amino, Chlor, Fluor, Alkyl, Alkoxy, Alkanoylamino oder Dialkylcarbamoyl substituiertes Benzyl, $R_4$ und $R_5$ für Alkyl mit obiger Bedeutung, $R_2$ für Methyl und $R_3$ für Isopropyl.

Das nachfolgende Beispiel erläutert die Erfindung. Temperaturangaben erfolgen in Celsiusgraden und sind unkorrigiert.

Beispiel

1,10'aβ-Dimethyl-9,10-dihydro-ergocristin

[Formel I: $R_1 = CH_2C_6H_5$, $R_2 = R_4 = R_5 = CH_3$, $R_3 = CH(CH_3)_2$]

5,08 g (0,04 Mol) Oxalylchlorid verdünnt mit 7 ml abs. Acetonitril werden innert 10 Minuten zu den auf −20° gekühlten 100 ml abs. Dimethylformamid zugetropft. Die gelbe Suspension wird nach weiteren 10 Minuten bei −20° mit 11,35 g (0,04 Mil) 1-Methyl-9,10-dihydrolysergsäure versetzt und 30 Minuten bei −10 bis 0° weitergerührt. Nach erneutem Abkühlen auf −20° werden bei derselben Temperatur 40 ml abs. Pyridin möglichst rasch zugegeben, gefolgt von 9,65 g (0,02 Mol) (2R,5S,10aS,10bS) - 2 - Amino - 2-isopropyl-5-benzyl-10b-hydroxy-10a-methyl-3,6-dioxo-octahydrooxazolo[3,2-a]pyrrolo[2,1-c]pyrazin-hydrochlorid erhalten, wie im folgenden unter e) beschrieben. Zur Beendigung der Reaktion wird 2 Stunden bei 0° gerührt, dann die Suspension auf −15° abgekühlt und mit 40 ml Citratpuffer pH = 4 so versetzt, dass die Temperatur von −15° nicht überschritten wird. Nun wird auf 2 N Sodalösung gegossen und mit Methylenchlorid extrahiert. Nach dem Trocknen der Lösungen und dem Entfernen de flüchtigen Bestandteile im Vakuum fällt kristallisiertes Rohprodukt an, welches zur Reinigung an der 10-fachen Menge Aluminiumoxid Aktivität II chromatographiert wird. Mit Methylenchlorid eluiert fast farbloses Produkt, welches nach Umkristallisation aus Methylenchlorid/Alkohol die Titelverbindung ergibt. Smp. 202—203° (Zers.); $[\alpha]_D^{20} = +2,9°$ (c = 1,6, Methylenchlorid).

Die als Ausgangsmaterial benötigte Verbindung wird auf folgendem Weg erhalten:

a) (2'S,3S,9S) - 2 - (2' - Benzyloxy - 2' - isopropyl - O - äthylmalonyl) - 1,4 - dioxo - 3 - benzyl - 9 - methyl - octahydropyrrolo[1,2-a]pyrazin

Eine Suspension von 67,8 g L-Phenylalanyl-L-α-methyl-prolin-lactam [$[\alpha]_D^{20} = -86°$ (c = 1, Aethanol)] in 40 ml abs. Pyridin und 35 ml abs. Dioxan werden auf 0° abgekühlt und dazu unter Rühren bei 0—5° innert 5 Minuten 74,8 g S-(+)-Isopropyl-benzyloxymalonsäure-monoäthyllesterchlorid zugegeben. Die weisse Suspension wird in 20 Minuten auf 60° erwärmt und 7 Stunden bei dieser Temperatur gehalten. Dann wird auf Eis gegossen, mit 2N HCl sauer gestellt und mit Aether extrahiert. Die Aetherextrakte werden mit Eisewasser, mit Natriumhydrogencarbonat-Lösung und nochmals mit Eiswasser gewaschen. Die ätherischen Lösungen werden mit $Na_2SO_4$ gut getrocknet und vom Lösungsmittel schonend befreit. Nach dem Trocknen im Hochvakuum verbleibt ein oranger Schaum.

b) (2R,5S,10aS,10bS) - 2 - Aethoxycarbonyl - 2 - isopropyl - 5 - benzyl - 10b - hydroxy - 10a-methyl - 3,6 - dioxo - octahydrooxazolo[3,2-a]pyrrolo[2,1-c]pyrazin

132 g der unter a) bezeichneten Verbindung werden in 1800 ml Alkohol mit 35 g Palladium auf Aktivkohle (10% Pd) als Katalysator bei 40—42° und Normaldruck während 20 Stunden hydriert. Nach beendeter Wasserstoffaufnahme wird vom Katalysator abfiltriert und das Lösungsmittel im Vakuum entfernt. Der Rückstand wird aus Isopropyläther/Methylenchlorid kristallisiert, wobei die reine Titelverbindung vom Smp. 105—107° anfällt; $[\alpha]_D^{20} = +48,3°$ (c = 1, Aethanol).

c) (2R,5S,10aS,10bS) - 2 - Carboxy - 2 - isopropyl - 5 - benzyl - 10b - hydroxy - 10a - methyl - 3,6 - dioxo - octahydro - oxazolo[3,2-a]pyrrolo[2,1-c]pyrazin

43 g der unter b) bezeichneten Titelverbindung werden bei Zimmertemperatur 6 Stunden in 105 ml 2N NaOH stehengelassen. Die klare Lösung wird mit 26,5 ml 4N HCl auf pH 7 gestellt und zweimal mit Essigester extrahiert. Die wässrige Phase wird mit 26,5 ml 4N HCl auf pH ca. 1 gestellt, im Eisbad abgekühlt, wobei die Titelverbindung auskristallisierte. Nach Filtration und Nachwaschen mit wenig Eiswasser wird 1 Mol Kristallwasser enthaltend die Titelverbindung gewonnen. Smp. 116—118° (Zers.); $[\alpha]_D^{20} = +25°$ (c = 1, Aethanol).

d) (2R,5S,10aS,10bS) - 2 - N - Benzyloxycarbonyl - amino - 2 - isopropyl - 5 - benzyl - 10b - hydroxy - 10a - methyl - 3,6 - dioxo - octahydro - oxazolo[3,2-a]pyrrolo[2,1-c]pyrazin

12,7 g Oxalylchlorid, verdünnt mit 20 ml abs. Chloroform, werden innert 10 Minuten zu einer auf —25° abgekühlten Mischung von 9,12 g abs. Dimethylformamid und 50 ml abs. Chloroform zugetropft. Die klare, farblose Lösung wird noch 10 Minuten bei ca. —10° nachgerührt, mit 21 g der unter c) bezeichneten Titelverbindung zusammen mit 50 ml abs. Chloroform versetzt, dann lässt man 10 Minuten bei —10 bis —15° reagieren, wobei eine klare Lösung von der unter c) bezeichneten Titelverbindung als Säurechlorid entsteht. Darauf werden unter kräftigem Rühren die in 40 ml Wasser gelösten 13 g Natriumazid in einem Guss zu der vorher auf —25° abgekühlten Säurechloridlösung zugegeben. Nach 3-minutigem, intensivem Rühren bei —5 bis 0° werden vorsichtig 250 ml 20-%ige Kaliumhydrogencarbonat-Lösung zugegeben, dreimal mit kaltem Chloroform extrahiert, die organischen Phasen mit Natriumsulfat gut getrocknet und die filtrierte, klare Lösung bei max. 25° Badtemperatur bis auf ein Volumen von ca. 250 ml eingeengt. Hierauf wird mit 10,8 g Benzylalkohol und 1 Tropfen konz. Salzsäure versetzt und während 40 Minuten unter Rückfluss gekocht. Die Reaktionslösung wird im Wasserstrahl-Vakuum und anschliessend im Hochvakuum bei 90° möglichst vollständig von flüchtigen Anteilen befreit und der Rückstand aus Essigester/Isopropyläther kristallisiert. Die Titelverbindung hat einen Smp. von 176—178° (Methylenchlorid/Essigester); $[\alpha]_D^{20} = +37°$ (c = 2, Aethanol).

e) (2R,5S,10aS,10bS) - 2 - Amino - 2 - isopropyl - 5 - benzyl - 10b - hydroxy - 10a - methyl - 3,6 - dioxo - octahydro - oxazolo[3,2-a]pyrrolo[2,1-c]pyrazin - hydrochlorid

40,6 g der unter d) erhaltenen Titelverbindung werden in einem Gemisch von 120 ml abs. Dimethylformamid, 275 ml abs. Tetrahydrofuran und 18,5 ml 5,6 N-Tetrahydrofuran-Salzsäurelösung in Gegenwart von 9 g Palladium-Aktivkohle-Katalysator (10% Pd) bei 20° und Normaldruck hydriert. Nach 10 Minuten intensivem Rühren und nach einer Wasserstoffaufnahme von 1,75 Liter ist die Hydrierung beendet. Der Katalysator wird abfiltriert und das farblose Filtrat im Vakuum bis zu einem viskosen Oel eingeengt. Das Oel wird in 40 ml abs. Tetrahydrofuran und 100 ml abs. Aether aufgenommen, worauf die Titelverbindung auskristallisiert. Die Kristalle werden abfiltriert, gewaschen mit einer Mischung von 1 Teil Tetrahydrofuran und 2 Teilen Aether, dann im Hochvakuum bei 20° getrocknet: farblose, rhombische Kristalle mit 1 Molekel Kristall-Dimethylformamid. Smp. 107—109° (Zers.); $[\alpha]_D^{20} = +19,8°$ (c = 1, Dimethylformamid).

**Patentansprüche**

1. Verbindungen der Formel I,

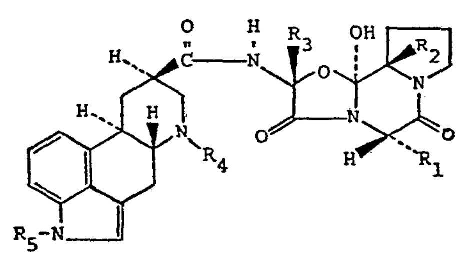

I

worin $R_1$ gegebenenfalls durch Hydroxy, Chlor, Fluor, Carbamoyl, Amino, Alkanoylamino mit 2 bis 5 Kohlenstoffatomen, Alkyl, Alkylamino, Alkylcarbamoyl oder Alkoxy mit 1 bis 4 Kohlenstoffatomen, Dialkylcarbamoyl oder Dialkylamino, wobei jede Alkylgruppe der beiden letzten Radikale 1 bis 4 Kohlenstoffatome enthält, substituiertes Benzyl, $R_2$, $R_4$ und $R_5$ Alkyl mit 1 bis 4 Kohlenstoffatomen und $R_3$ verzweigtes Alkyl mit 3 bis 4 Kohlenstoffatomen bedeuten, und ihre Säureadditionssalze.

2. Verfahren zur Herstellung von Verbindungen der Formel I, worin $R_1$, $R_2$, $R_3$, $R_4$ und $R_5$ die im Anspruch 1 angegebene Bedeutung besitzen, nach Anspruch 1 dadurch gekennzeichnet, dass man ein Säureadditionssalz einer Verbindung der Formel II,

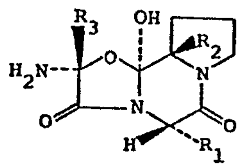

II

worin $R_1$, $R_2$ und $R_3$ obige Bedeutung haben, mit einem reaktiven Säurederivat einer Verbindung der Formel III,

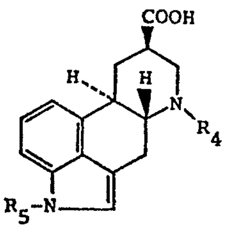

III

worin $R_4$ und $R_5$ obige Bedeutung haben, kondensiert und die so erhaltenen Verbindungen der Formel I in Form der Basen oder von Säureadditionssalzen gewinnt.

3. Heilmittel, enthaltend eine Verbindung der Formel I nach Anspruch 1 oder ein physiologisch verträgliches Säureadditionssalz mit einem in der Pharmazie gebräuchlichen Träger oder Verdünnungsmittel.

4, 1,10'aβ-Dimethyl-9,10-dihydro-ergocristin nach Anspruch 1 in Form der Base oder Säureadditionssalze.

5. Eine Verbindung der Formel I nach Anspruch 1, worin $R_1$ für Benzyl oder durch Hydroxy, Amino, Chlor, Fluor, Alkyl, Alkoxy, Alkanoylamino oder Dialkylcarbamoyl substituiertes Benzyl, $R_4$ und $R_5$ für Alkyl mit obiger Bedeutung stehen und $R_2$ für Methyl und $R_3$ für Isopropyl stehen, und ihre Säureadditionssalze.

6. Eine Verbindung gemäss Anspruch 1 in Form des Base oder eines pharmazeutisch verträglichen Säureadditionssalzes zur Anwendung als Heilmittel.

7. Eine Verbindung gemäss Anspruch 6 zur Anwendung als Prolactinsekretionsförderer.

## Claims

1. Compounds of formula I,

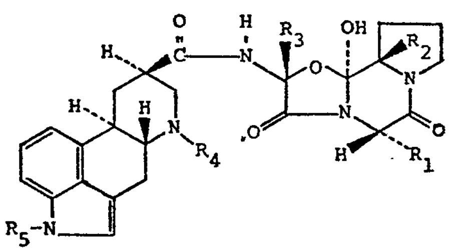

I

wherein $R_1$ represents benzyl optionally substituted by hydroxy, chlorine, fluorine, carbamoyl, amino, alkanoylamino with 2 to 5 carbon atoms, alkyl, alkylamino, alkylcarbamoyl or alkoxy with 1 to 4 carbon atoms, dialkylcarbamoyl or dialkylamino, whereby each alkyl group of the two last radicals contains 1 to 4 carbon atoms, $R_2$, $R_4$ and $R_5$ represent alkyl with 1 to 4 carbon atoms and $R_3$ represents branched alkyl with 3 to 4 carbon atoms, and their acid addition salts.

2. Process for the production of compounds of formula I, wherein $R_1$, $R_2$, $R_3$, $R_4$ and $R_5$ have the meanings given in claim 1, in accordance with claim 1, characterised in that an acid addition salt of a compound of formula II,

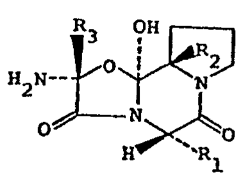

II

wherein $R_1$, $R_2$ and $R_3$ have the meanings given above, is condensed with a reactive acid derivative of a compound of formula III,

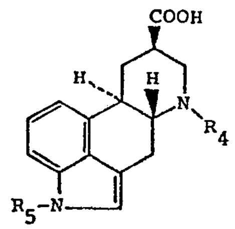

III

wherein $R_4$ and $R_5$ have the meanings given above, and the compounds of formula I thus obtained are recovered in the form of the bases or of acid addition salts.

3. Pharmaceutical composition containing a compound of formula I according to claim 1 or a physiologically acceptable acid addition salt with a pharmaceutically applicable carrier or diluent.

4. 1,10'aβ-Dimethyl-9,10-dihydroergocristine according to claim 1 in the form of a base or acid addition salts.

5. A compound of formula I according to claim 1, wherein $R_1$ represents benzyl or benzyl substituted by hydroxy, amino, chlorine, fluorine, alkyl, alkoxy, alkanoylamino or dialkylcarbamoyl and $R_4$ and $R_5$ represent alkyl with the above significance and $R_2$ represents methyl and $R_3$ represents isopropyl and its acid addition salts.

6. A compound according to claim 1 in the form of the base or of a pharmaceutically tolerable acid addition salt for use as a pharmaceutical.

7. A compound according to claim 6 for use as a prolactin secretion stimulator.

**Revendications**

1. Composé de formule I,

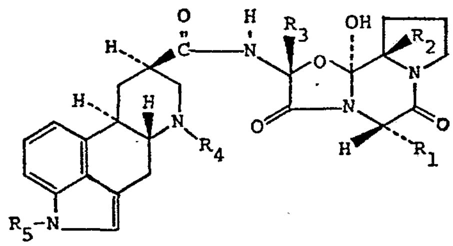

(I)

dans laquelle

$R_1$ signifie un groupe benzyle éventuellement substitué par des radicaux hydroxy, chloro, fluoro, carbamoyle, amino, alcanoylamino contenant de 2 à 5 atomes de carbone, alkyle, alkylamino, alkylcarbamoyle ou alcoxy contenant de 1 à 4 atomes de carbone, dialkylcarbamoyle ou dialkylamino, chaque groupe alkyle des deux derniers radicaux contenant de 1 à 4 atomes de carbone, $R_2$, $R_4$ et $R_5$ signifient un groupe alkyle contenant de 1 à 4 atomes de carbone et $R_3$ signifie un groupe alkyle ramifié contenant 3 ou 4 atomes de carbone, et leurs sels d'addition d'acides.

2. Procédé de préparation de composés de formule I, dans laquelle $R_1$, $R_2$, $R_3$, $R_4$ et $R_5$ ont la signification donnée à la revendication 1, selon la revendication 1, caractérisé en ce que l'on condense un sel d'addition d'acides d'un composé de formule II,

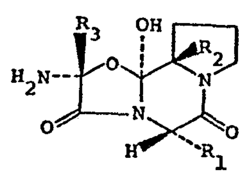

(II)

dans laquelle $R_1$, $R_2$ et $R_3$ ont la signification donnée ci-dessus, avec un dérivé d'acide réactif d'un composé de formule III,

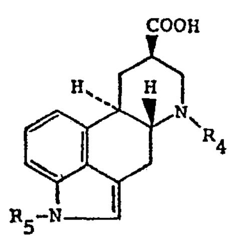

(III)

dans laquelle $R_4$ et $R_5$ ont la signification donnée ci-dessus, et on récupère les composés de formule I ainsi obtenus, sous forme de bases ou de sels d'addition d'acides.

3. Médicaments contenant un composé de formule I selon la revendication 1 ou un sel d'addition d'acides acceptable du point de vue physiologique, en associaton avec un véhicule ou un diluant utilisés en pharmacie.

4. 1,10'aβ-diméthyl-9,10-dihydro-ergocristine selon la revendication 1, sous forme de base ou de sels d'addition d'acides.

5. Un composé de formule I selon la revendication 1, caractérisé en ce que $R_1$ signifie un groupe benzyle ou un groupe benzyle substitué par des radicaux hydroxy, amino, chloro, fluoro, alkyle, alcoxy, alcanoylamino ou dialkylcarbamoyle, $R_4$ et $R_5$ représentent un groupe alkyle ayant la signification donnée ci-dessus et $R_2$ signifie un groupe méthyle et $R_3$ un groupe isopropyle, et leurs sels d'addition d'acides.

6. Un composé selon la revendication 1 sous forme de base ou d'un sel d'addition d'acides acceptable du point de vue pharmaceutique, pour l'utilisation comme médicament.

7. Un composé selon la revendication 6 pour l'utilisation comme stimulant de la sécrétion de la prolactine.